# EUROPÄISCHE PATENTSCHRIFT

(11) **EP 3 253 453 B1**
(45) Veröffentlichungstag und Bekanntmachung des Hinweises auf die Patenterteilung: **18.09.2019**
(21) Anmeldenummer: 16702412.4
(22) Anmeldetag: 01.02.2016
(51) Int. Cl.: A61N 5/06, A01K 29/00, A23C 9/158, A61K 35/20, A01K 1/12, A01K 1/00

(54) **VERFAHREN ZUR HERSTELLUNG VON MILCH MIT HOHEM GEHALT AN NATIVEM VITAMIN D**
METHOD FOR THE PRODUCTION OF MILK WITH HIGH NATIVE VITAMIN D CONTENT
PROCÉDÉ DE FABRICATION DE LAIT À HAUTE TENEUR EN VITAMINE D NATIVE

(30) Priorität: 04.02.2015 EP 15153835
(43) Veröffentlichungstag der Anmeldung: 13.12.2017
(73) Patentinhaber: Raichle, Marianne, 80802 München (DE)
(72) Erfinder: GNANN, Tony, 80802 München (DE)
(74) Vertreter: Held, Stephan
(86) Internationale Anmeldenummer: PCT/EP2016/052031
(87) Internationale Veröffentlichungsnummer: WO 2016/124521

(56) Entgegenhaltungen:
- EP-A1- 1 970 423
- EP-A1- 2 573 799
- WO-A1-84/00693
- WO-A1-2014/184277
- CN-A- 104 168 953
- DE-A1-102005 059 518

## Beschreibung

Das Verfahren betrifft ein Verfahren zur Herstellung von Milch mit hohem Gehalt an nativem Vitamin D3.

Vitamin D nimmt bei Menschen und Tieren eine zentrale Stellung in der Regulation des Calcium- und Phosphat-Stoffwechsels ein. Vitamin D-Mangel führt zur erheblichen Störungen im Knochenbau und kann bei Kindern zu Rachitis und bei Erwachsenen zu Osteomalazie führen. Ausreichend Vitamin D erzeugt eine starke Immunisierung und kann gegen einige chronische Krankheiten präventiv wirken.

Vitamin-D-Mangel kann bei Menschen in der Erdnordhälfte insbesondere im Herbst und Winter eintreten. Außer in Lebertran und Fettfischen weisen Lebensmittel nur geringe Vitamin D-Gehalte auf. Die künstliche Anreicherung von Vitamin D in Lebensmitteln durch Zugabe von synthetisch erzeugtem Vitamin D ist zumindest in Deutschland nicht erlaubt. Synthetisches Vitamin D kann synthetisch hergestellt oder aus natürlichen Quellen wie Fischleberölen gewonnen werden.

Gemäß der europäischen Verordnung (EU) 1169/2011 wird eine Tagesdosis an Vitamin D von 5 µg empfohlen. Der Gehalt an Vitamin D3 in handelsüblicher Kuhmilch beträgt gemäß aktuellem Bundeslebensmittelschlüssel (Version 3.01) 0,1 µg/100 g, was nur etwa 2% der empfohlenen Tagesdosis ausmacht. Daraus ergibt sich, dass die heutzutage erhältliche Milch nicht geeignet ist, einen signifikanten Beitrag zum Vitamin D-Bedarf zu liefern.

Bei der heute für größere Tierherden üblichen Laufstallhaltung für laktierende Säugetiere, insbesondere Milchkühe, können sich die Tiere im Stall frei bewegen und sowohl tagsüber wie auch nachts ihre Liege-, Fress- und Melkplätze frei aufsuchen. Die Ställe werden tagsüber meist mittels Leuchten beleuchtet und sind nachts mit meist weißfarbiger Notbeleuchtung ausgestattet.

Die Veröffentlichung der Europäischen Patentanmeldung EP2573799 A1 offenbart ein Verfahren zur Herstellung von Milch mit hohem nativem Vitamin D3-Gehalt umfassend die Bestrahlung eines oder mehrerer laktierende Tiere in einem überdachten Aufenthaltsraum mit mindestens einer Leuchte umfassend eine Vollspektrumlampe, die UV-A-Strahlung und UV-B-Strahlung emittiert, und das Melken der Tiere.

Die Aufgabe der vorliegenden Erfindung bestand in der Bereitstellung eines Verfahrens zur Herstellung von Milch mit erhöhtem Vitamin D-Gehalt. Dabei soll der natürliche Vitamin D-Gehalt der Milch erhöht werden. Das Verfahren soll insbesondere auch für eine erhöhte Anzahl von Tieren geeignet sein.

Diese Aufgaben konnten überraschenderweise durch ein Verfahren gemäß Anspruch 1 zur Herstellung von Milch gelöst werden, bei dem ein oder mehrere laktierende Tiere in einem überdachtem Aufenthaltsraum, bevorzugt einem Stall, mit mindestens einer Leuchte umfassend eine Lampe, die UV-A-Strahlung und UV-B-Strahlung emittiert, bestrahlt werden und die Tiere gemolken werden.

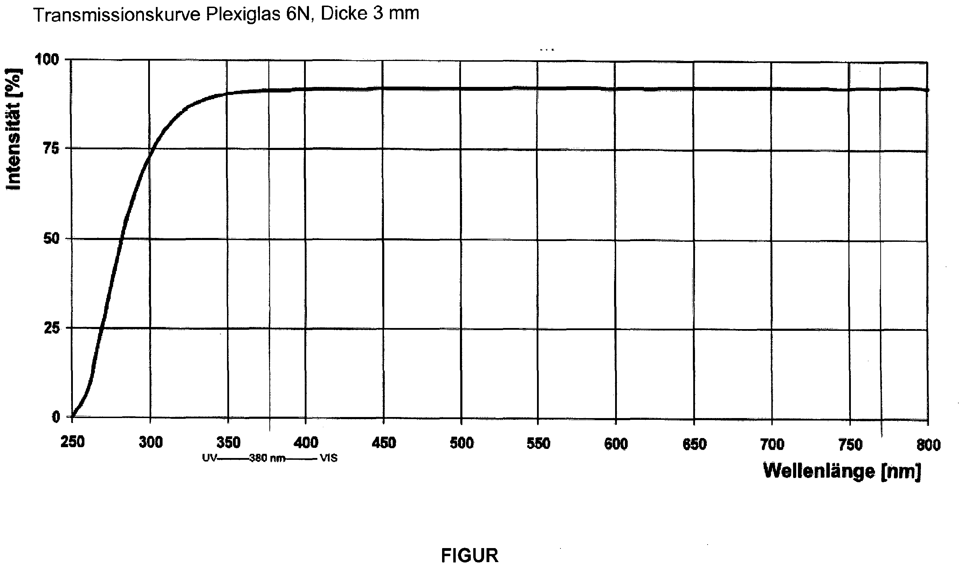

Durch das erfindungsgemäße Verfahren, das in dem Anspruch 1 definiert wird, konnte überraschenderweise Milch mit einem sehr hohen Gehalt an Vitamin D3 von mehr als 0,5 µg/100 ml und sogar im Bereich von 1,5 bis über 4 µg/100 ml produziert werden. Mit dieser Milch kann beim Menschen ein Großteil des Vitamin D-Tagesbedarfs oder sogar der gesamte Vitamin D-Bedarf gedeckt werden. Bei der erfindungsgemäß hergestellten Milch handelt es sich weiterhin um ein gewöhnliches Lebensmittel, da das darin enthaltene Vitamin D3 natives Vitamin D3 ist.
Die Figur zeigt eine Transmissionskurve eines speziellen Plexiglases, welches für die Lampenabdeckung geeignet ist.

Natives Vitamin D3 in der Milch wird in dem laktierenden Tier auf natürliche Weise gebildet. Vitamin D3, das der Milch nach dem Melken zugesetzt wird, ist kein natives Vitamin D3. Unter Vitamin D wird im Folgenden Vitamin D3 verstanden.

Laktierende Tiere sind weibliche Säugetiere, die Milch abgeben, wobei Schafe, Kühe und Ziegen besonders geeignet sind. Bevorzugt handelt es sich bei den laktierenden Tieren um Kühe. Es werden ein oder mehrere laktierende Tiere in dem überdachten Aufenthaltsraum, insbesondere einem Stall, gehalten, bevorzugt mindestens 10, bevorzugter mindestens 50 oder 100 oder sogar mehr als 200 Tiere.

Der überdachte Aufenthaltsraum ist vorzugsweise ein Stall. Der überdachte Aufenthaltsraum kann auch einfach eine überdachte Behausung oder ein Unterstand sein. Der überdachte Aufenthaltsraum ist bevorzugt ein Stall in Form eines geschlossenen Gebäudes.

Eine Leuchte ist eine Vorrichtung, die als Lichtquelle eine Lampe aufweist und zur Beleuchtung dient. Im Rahmen dieser Erfindung wird die Bestrahlung mit UV-Licht auch als Beleuchtung verstanden. Eine Lampe wird auch als Leuchtmittel bezeichnet. Erfindungsgemäß können eine oder mehrere Leuchten zur Beleuchtung des überdachten Aufenthaltsraums bzw. zur Bestrahlung der Tiere verwendet werden. Die erfindungsgemäß verwendete Leuchte umfasst eine Lampe, die UV-A-Strahlung und UV-B-Strahlung emittiert, d.h. die Lampe weist ein Emissionsspektrum auf, das Anteile im UV-A-Bereich und im UV-B-Bereich aufweist.

Unter Emissionsspektrum der Lampe wird hier die emittierte Strahlung im Wellenlängenbereich von 200 nm bis 780 nm verstanden. Dabei gilt gemäß DIN 5031-7 folgende Unterteilung:

| | |
|---|---|
| sichtbares Licht (VIS) | 380 bis 780 nm |
| UV-A-Strahlung | 315 bis 380 nm |
| UV-B-Strahlung | 280 bis 315 nm |
| UV-C-Strahlung | 200 bis 280 nm |

Die erfindungsgemäß verwendeten Lampen weisen im Emissionsspektrum sowohl Anteile im UV-A-Bereich als auch Anteile im UV-B-Bereich auf. Während üblicherweise zur Beleuchtung eingesetzte Lampen ein Emissionsspektrum besitzen, das manchmal auch Anteile im UV-A-Bereich aufweist, sind Anteile im UV-B-Bereich im allgemeinen nicht vorhanden.

Der Anteil an Strahlung im UV-B-Bereich im Emissionsspektrum der Lampe beträgt mindestens 0,2% und besonders bevorzugt mindestens 0,5%. Der Anteil an Strahlung im UV-B-Bereich im Emissionsspektrum der Lampe beträgt weniger als 5%, bevorzugter weniger als 4%, besonders bevorzugt weniger als 2%. Der Anteil bezieht sich auf den prozentualen Anteil der Bestrahlungsstärke der Lampe im UV-B-Bereich bezogen auf die gesamte Bestrahlungsstärke der Lampe im Emissionsspektrum im Wellenlängenbereich von 200 bis 780 nm.

Der Anteil an Strahlung im UV-A-Bereich im Emissionsspektrum der Lampe kann z.B. mindestens 0,5%, bevorzugt mindestens 1% und besonders bevorzugt mindestens 4,5% betragen. In der Regel ist es bevorzugt, wenn der Anteil an Strahlung im UV-A-Bereich im Emissionsspektrum der Lampe weniger als 20%, bevorzugt weniger als 15% beträgt. Der Anteil bezieht sich auf den prozentualen Anteil der Bestrahlungsstärke der Lampe im UV-A-Bereich bezogen auf die gesamte Bestrahlungsstärke der Lampe im Emissionsspektrum im Wellenlängenbereich von 200 bis 780 nm.

Die Lampe weist auch sichtbares Licht im Emissionsspektrum auf, z.B. einen Anteil an Strahlung im VIS-Bereich im Emissionsspektrum der Lampe von mindestens 50%, bevorzugt mindestens 70% und besonders bevorzugt mindestens 80%. Der Anteil bezieht sich auf den prozentualen Anteil der Bestrahlungsstärke der Lampe im VIS-Bereich bezogen auf die gesamte Bestrahlungsstärke der Lampe im Emissionsspektrum im Wellenlängenbereich von 200 bis 780 nm. Die Lampe strahlt vorzugsweise weißes Licht aus.

Lampenspezifische Parameter wie die Bestrahlungsstärke (Einheit W/m²) sind in der DIN 5031 definiert. Die spektrale Bestrahlungsstärke gibt an, wie viel Bestrahlungsstärke in einem bestimmten Wellenlängenbereich vorliegt. Die spektralen Bestrahlungsstärken bzw. deren prozentualen Anteile können z.B. mit einem Spektrometer oder Spektroradiometer bestimmt werden. In der Regel sind prozentuale Angaben zu den spektralen Bestrahlungsstärken in den Datenblättern der Lampen enthalten.

Der Farbwiedergabeindex (englisch Colour Rendering Index, CRI) ist eine Kennzahl auf einer Skala von 0 bis 100, mit der die Qualität der Farbwiedergabe von Lichtquellen beschrieben wird. Die erfindungsgemäß verwendete Lampe weist vorzugsweise einen Farbwiedergabeindex von größer 40, insbesondere größer 70 und besonders bevorzugt größer 80 auf. Der Farbwiedergabeindex kann gemäß Commission Internationale de l'Eclairage, Method of Measuring and Specifying Colour Rendering Properties of Light Sources, Publ. CIE 13.3-1995, 1995, bestimmt werden.

Als Lampe kann jede Lampe, die UV-A-Strahlung und UV-B-Strahlung emittiert, verwendet werden. Die Lampe ist eine Vollspektrumlampe. Vollspektrumlampen haben in der Regel einen Farbwiedergabeindex von größer 70 und bevorzugt von größer 80. Die Farbtemperatur von Vollspektrumlampen beträgt in der Regel mindestens 5000 K.

UV-Lampen können eingesetzt werden, sind aber weniger bevorzugt. Wegen des hohen UV-Anteils bei UV-Lampen ist darauf zu achten, dass die Tiere keine zu hohe Bestrahlungsdosis erhalten. Dies kann z.B. durch eine geringere Bestrahlungsdauer erreicht werden. Bei Einsatz von UV-Lampen ist ferner in der Regel der Einsatz zusätzlicher gewöhnlicher Lampen zur Beleuchtung mit sichtbarem Licht erforderlich. Dies ist z.B. bei Einsatz von Vollspektrumlampen, die UV-A-Strahlung und UV-B-Strahlung emittieren können, nicht erforderlich, da diese gleichzeitig die Beleuchtung mit sichtbarem Licht bereitstellen.

Die Lampe kann z.B. eine Gasentladungslampe, eine Leuchtstofflampe, eine Halogen-Metalldampflampe oder eine LED-Lampe sein. LED (light emitting diode) werden auch als Leuchtdioden bezeichnet. LED-Lampen, die Anteile im UV-A-Bereich und UV-B-Bereich emittieren, sind gut geeignet. Bevorzugt handelt es sich bei den LED-Lampen, die Anteile im UV-A-Bereich und UV-B-Bereich emittieren, um weiße LED-Lampen, d.h. Lampen, die auch weißes Licht emittieren. Vorteilhaft bei LED-Lampen ist auch der geringe Energieverbrauch. Die Gasentladungslampen können Niederdruckentladungslampen oder Hochdruckentladungslampen sein. Leuchtstofflampen sind in der Regel Niederdruckentladungslampen. Halogen-Metalldampflampen sind in der Regel Hochdruckentladungslampen.

Bei der Lampe handelt es sich besonders bevorzugt um eine Leuchtstofflampe. Leuchtstoffe generieren in Leuchtstofflampen über physiko-chemische Prozesse sichtbare Strahlung/Licht (380 bis 780 nm) bzw. UV-Strahlung im Wellenlängenbereich von ca. 280 - 380 nm und gegebenenfalls auch unter 280 nm. Leuchtstoffe sind in der Regel anorganische Verbindungen, üblicherweise Salze oder in einigen Fällen auch Oxide, die hochrein sind, aber zusätzlich sogenannte Fehldotierungen bzw. Aktivatoren besitzen. Die chemische Struktur kombiniert mit Fehlstellen bestimmt die Emissionseigenschaften der Leuchtstoffe.

Gemäß der üblichen Nomenklatur wird für einen Leuchtstoff die eingesetzte Verbindung und der Aktivator bzw. das Dotierungsmittel angegeben, die durch einen Doppelpunkt getrennt werden. Strontium-Borophosphat:Eu ist z.B. mit Europium dotiertes Strontium-Borophosphat.

Übliche Leuchtstofflampen sind sogenannte Dreibandenlampen, die durch Mischen von rot, grün und blau emittierender Strahlung weißes Licht generieren. Es ist bekannt, dass grün emittierende Leuchtstoffe auch im UV-A-Bereich emittieren und eine Hg-Emission bei 365 nm existiert. Solche Dreibandenlampen emittieren daher auch im UV-A-Bereich (ca. 2 bis 4%), nicht aber im UV-B-Bereich. Der Anteil im VIS-Bereich beträgt in der Regel etwa 96 bis 98 %.

Werden in Leuchtstofflampen für bestimmte Anwendungen UV-emittierende Leuchtstoffe verwendet, entsteht eine durch die Leuchtstoffe bedingte Emission, die jedoch durch die wellenlängenabhängige Glastransmission des Lampenkolbens im kürzerwelligen UV-Bereich stärker absorbiert wird. Sollen Leuchtstofflampen mit einem Emissionsspektrum im VIS-, im UV-A- und UV-B-Bereich generiert werden, ist eine Mischung von Leuchtstoffen notwendig, die im Licht-, UV-A- und UV-B-Bereich emittieren. Leuchtstoffe für UV-B sind bekannt. Die Leuchtstofflampe kann z.B. Leuchtstoffe ausgewählt aus SrAl₁₂O₁₉:Ce, LaB₃O₆:Bi,Gd oder LaPO₄:Ce umfassen.

Ein Beispiel für eine geeignete Leuchtstofflampe ist z.B. eine Leuchtstofflampe, die Strontium-Borophosphat:Eu (SBPE), Strontium-Magnesium-Phosphat:Sn (SMS), Barium-Magnesium-Aluminat:Ce (BAC) und gegebenenfalls Lanthan-Phosphat: Ce (LAP) als Leuchtstoffe umfasst. Eine Leuchtstofflampe mit einer Mischung von ca. 36 % SBPE, ca. 50 % SMS, ca. 13 % BAC und max. 1 % LAP in Kombination mit einer bekannten Glastransmission erzeugt z.B. eine Bestrahlungsstärke, die sich wie folgt zusammensetzt: VIS-Anteil (380-780 nm): max. 90 %, UV-A-Anteil (315-380 nm): max. 10 %, UV-B-Anteil (280-315nm): max. 1 % und UV-C-Anteil (200-280 nm): 0 %.

Der Einsatz der Leuchte mit einer Lampe, die UV-A-Strahlung und UV-B-Strahlung emittiert, ergibt eine durch das Lichtregime gesteuerte Stimulierung der Vitamin-D3-Sekretion über die Haut der Tiere. Die Folge ist eine erhöhte Vitamin-D3-Konzentration im Blutplasma. Der Vitamin-D3-Gehalt im Blutplasma wiederum korreliert mit der Vitamin-D3-Konzentration in der Milch.

In Aufenthaltsräumen für Nutztiere, insbesondere in Ställen, ist durch die Lagerung und den Umgang mit leicht entzündlichen Stoffen wie Futtermittel, Heu, Stroh etc. generell mit einer erheblichen Feuergefährdung durch Staub oder Fasern zu rechnen. Es dürfen daher aufgrund von staatlichen Normen in der Regel nur Leuchten eingesetzt werden, die einen hohen Schutzgrad aufweisen und darüber hinaus zusätzlich mit dem Zeichen gekennzeichnet sind. In Tierställen ist die Luft zusätzlich mehr oder weniger mit hoher Feuchtigkeit und Ammoniak belastet, was an ungeeigneten Leuchten schnell zu Korrosionsschäden und damit zum Ausfall und zur Zerstörung führt. In Aufenthaltsräumen von Nutztieren, insbesondere in Ställen, sind deshalb zumindest in Deutschland Leuchten mit der Schutzart IP 65 vorgeschrieben.

Die IP-Schutzarten gemäß DIN 60529 legen fest, inwieweit eine Lampe gegen Fremdkörper und Wasser geschützt ist. Die Schutzart IP 65 bedeutet z.B. das die Leuchte staubdicht und gegen Strahlwasser geschützt ist.

Der Schutz der Lampe kann z.B. durch ein Gehäuse und/oder eine Lampenabdeckung, die manchmal auch als Abdeckwanne bezeichnet wird, erfolgen. Die Lampenabdeckung muss naturgemäß für von der Lampe emittiertes Licht durchlässig sein. Üblich sind Leuchten mit Abdeckwannen bzw. Lampenabdeckungen aus Glas oder Kunststoff. Diese Lampenabdeckungen sind zwar wie gewünscht für sichtbares Licht durchlässig, reduzieren aber die Transmission von UV-Strahlen, sofern vorhanden, insbesondere im UV-B-Bereich, fast vollständig.

So ist normales Glas (Natron-Kalk-Glas), insbesondere Fensterglas, für UV-Strahlung unterhalb von 320 nm nicht durchlässig. Borosilikatglas ist dagegen für UV-Strahlung bis hinunter auf etwa 290 nm durchlässig. Quarzglas ist bis hinunter auf etwa 200 nm durchlässig. Kunststoffe sind häufig empfindlich gegen UV-Strahlung. Je nach Ausführungsform können einige Kunststoffmaterialien auch UV-durchlässig ausgebildet sein. So gibt es Ausführungsformen von Polymethylmethacrylat (PMMA), die UV-durchlässig sind. Weitere UV-durchlässige Kunststoffe sind Polymethylpenten, Fluoriertes-Ethylen-Propylen (FEP) oder Polytetrafluorethylen (PTFE).

Kunststoffe enthalten häufig UV-Stabilisatoren. Die UV-Durchlässigkeit von bestimmten Kunststoffen kann z.B. durch Art und Menge der eingesetzten UV-Stabilisatoren eingestellt werden. UV-durchlässige Kunststoffe, z.B. UVdurchlässiges Plexiglas, sind im Handel erhältlich.

Die Leuchte umfasst in einer bevorzugten Ausführungsform eine Lampenabdeckung, die für UV-B-Strahlung mit einer Wellenlänge von 300 nm durchlässig ist. Der Transmissionsgrad der Lampenabdeckung bei einer Wellenlänge von 300 nm beträgt dabei z.B. mindestens 20%, bevorzugt mindestens 40%, besonders bevorzugt mindestens 50% und am meisten bevorzugt mindestens 60%.

In einer bevorzugten Ausführungsform umfasst die Leuchte eine Lampenabdeckung, die für UV-B-Strahlung der Lampe durchlässig ist, wobei die Lampenabdeckung vorzugsweise eine Durchlässigkeit von mindestens 20%, bevorzugt mindestens 40%, besonders bevorzugt mindestens 60%, für die von der Lampe emittierte UV-B-Strahlung aufweist.

Die Lampenabdeckung ist bevorzugt aus Quarzglas, Borosilikatglas, Polymethylmethacrylat (PMMA oder Plexiglas), Polymethylpenten, Fluoriertes-Ethylen-Propylen (FEP) oder Polytetrafluorethylen (PTFE) gebildet. Das Material der Lampenabdeckung kann naturgemäß wie üblich Zusatzstoffe oder Additive enthalten.

In einer bevorzugten Ausführungsform umfasst die Leuchte ein Gehäuse und eine Lampenabdeckung, wobei zwischen dem Gehäuse der Leuchte und der Lampenabdeckung eine Dichtung angeordnet ist. Die Dichtung kann z.B. aus Silicon, Polyurethan oder Gummi, z.B. EPDM (Ethylen-Propylen-Dien), sein, wobei Polyurethan bevorzugt ist. Durch die Dichtung kann das Eindringen von Fremdstoffen in die Leuchte vermieden oder deutlich reduziert werden. Die Dichtung, z.B. eine Dichtung aus Polyurethan, ist vorzugsweise eine Dichtung, auf die Talkum, gewöhnlich als Pulver, aufgebracht ist. Das Talkum kann z.B. einfach als Pulver auf die Dichtung gerieben oder gestrichen werden oder man verwendet eine Masse aus Talkumpulver und Wasser für den Auftrag.

Es ist bekannt, dass Dichtungsmaterial durch UV-B-Bestrahlung angegriffen werden kann. So hat sich in durchgeführten Versuchen gezeigt, dass bei Betrieb der erfindungsgemäßen Leuchten, die eine Dichtung enthielten, mit der Zeit eine Schädigung der Dichtung zu beobachten war, was die Dichtheit der Leuchte und die Ablösbarkeit der Abdeckwanne, z.B. für Instandhaltungsarbeiten, beeinträchtigt. Eine solche Schädigung der Dichtung trat nicht auf, wenn eine Dichtung mit darauf aufgebrachtem Talkum verwendet wurde.

Ein oder mehrere laktierende Tiere werden in einem überdachten Aufenthaltsraum, mit mindestens einer Leuchte umfassend eine Lampe, die UV-A-Strahlung und UV-B-Strahlung emittiert, bestrahlt. Hierfür wird der überdachte Aufenthaltsraum mit der oder den Leuchten beleuchtet. Die Anzahl und die Verteilung der Leuchten im überdachten Aufenthaltsraum hängen z.B. von der Art und Größe der überdachten Aufenthaltsräume, der gewünschten Beleuchtungsintensität und der Art der Leuchten ab. Es können die für die Beleuchtung von Ställen üblichen Lichtprogramme verwendet werden.

Die Dauer der Bestrahlung der laktierenden Tiere in einem überdachten Aufenthaltsraum, insbesondere einem Stall, mit mindestens einer Leuchte umfassend eine Lampe, die UV-A-Strahlung und UV-B-Strahlung emittiert, kann in breiten Bereichen variieren und hängt z.B. von dem UV-Anteil der Lampe und der Bestrahlungsstärke ab.
Die Halbwertszeit von Vitamin D im Organismus ist relativ groß. Es werden Halbwertszeiten von etwa 19 Tagen angegeben. Der Vitamin D-Gehalt sinkt daher nur allmählich, wenn die erfindungsgemäße Bestrahlung über einen gewissen Zeitraum ausgesetzt wird. Daraus folgt, dass durchaus längere Unterbrechungen von Tagen oder sogar Wochen zwischen den Bestrahlungsintervallen möglich sind und dennoch Milch mit erhöhtem Vitamin D3-Gehalt erhalten werden kann. Es ist daher eine diskontinuierliche Bestrahlung möglich, bei der die zugeführte UV-Dosis über zwei oder mehr voneinander getrennten Tage oder Wochen durchgeführt wird.

Vorteilhafterweise beträgt die Dauer der Bestrahlung mit der erfindungsgemäß verwendeten Leuchte pro Woche z.B. mindestens 6 Stunden, bevorzugt mindestens etwa 20 Stunden, bevorzugter mindestens etwa 50 Stunden. Sie kann pro Woche z.B. weniger als 155 Stunden, bevorzugt weniger als 135 Stunden betragen. Die Phasen der Bestrahlung können relativ gleichmäßig auf die Tage der Woche verteilt oder auf eine oder mehrere Tage konzentriert werden, wobei eine relativ gleichmäßige Verteilung auf die Tage der Woche bevorzugt ist.

In einer bevorzugten Ausführungsform werden die laktierenden Tiere pro Tag z.B. mindestens 1 Stunde, z.B. 6 bis 22 Stunden, zweckmäßigerweise etwa 8 bis 20 Stunden pro Tag mit der erfindungsgemäß verwendeten Leuchte bestrahlt. Die tägliche Bestrahlung kann vorzugsweise an einem Stück durchgeführt werden, es ist aber auch eine diskontinuierliche Bestrahlung möglich, bei der die zugeführte UV-Dosis über zwei oder mehr voneinander getrennten Zeitintervallen durchgeführt wird. Auch bei einem Tagesrhythmus mit täglicher Bestrahlung ist es möglich, die Bestrahlung an manchen Tagen auszusetzen. Auch in diesem Fall wird der Vitamin-D-Gehalt nicht wesentlich beeinträchtigt.
Die vorstehend genannten Bereiche für die Bestrahlungsdauer sind insbesondere dann zweckmäßig, wenn als Lampe eine Vollspektrumlampe verwendet wird. Bei Einsatz einer UV-Lampe ist wie vorstehend erläutert der UV-B-Anteil deutlich höher, so dass die Dauer der Bestrahlung mit einer Leuchte umfassend eine UV-Lampe in der Regel deutlich geringer sein sollte, z.B. mindestens 0,5 Stunden pro Woche, z.B. 3 Stunden bis 15 Stunden pro Woche oder mindestens 0,1 Stunden pro Tag, z.B. 0,5 bis 2 Stunden pro Tag. Die vorstehenden Angaben bezüglich Unterbrechungen und kontinuierlichen oder diskontinuierlichen Bestrahlungen gelten entsprechend.

Wie bereits ausgeführt emittiert die Leuchte auch sichtbares Licht, bevorzugt weißes Licht, so dass die Leuchte auch der Beleuchtung des überdachten Aufenthaltsraums mit sichtbarem Licht dient. Die Leuchte wird daher bevorzugt auch zur Beleuchtung des überdachten Aufenthaltsraums während der Tagphase verwendet. Es ist aber auch eine Verwendung in der Nachtphase und eine Verwendung sowohl in der Nachtphase als auch in der Tagphase möglich.
Die Beleuchtungsstärke, die durch die erfindungsgemäß verwendete Leuchte erhalten wird, kann z.B. mehr als 80 lux, bevorzugt mehr als 100 lux und bevorzugter mehr als 250 lux betragen. Die Beleuchtungsstärke kann z.B. bis zu 500 lux oder auch mehr betragen. Die Beleuchtungsstärke kann mit üblichen Luxmetern gemessen werden. Die angegebene Beleuchtungsstärke bezieht sich auf eine Messung in einer Höhe von etwa 1,50 m über dem Boden des überdachten Aufenthaltsraums.

Der Tagesrhythmus der laktierenden Tiere kann zweckmäßigerweise in eine Tagphase unter einem Lichtregime und eine Nachtphase unter einem anderen Lichtregime unterteilt werden. Die bei der Stallhaltung von laktierenden Tieren üblichen Lichtprogramme können ohne weiteres übernommen werden, außer dass die erfindungsgemäß verwendete Leuchte eingesetzt wird, z.B. um die üblicherweise zur Beleuchtung mit sichtbarem Licht eingesetzten Leuchten zu ersetzen.

In einer bevorzugten Ausführungsform des erfindungsgemäßen Verfahrens wird der Tagesrhythmus der Tiere in eine Tagphase unter einem ersten Lichtregime und in eine Nachtphase unter einem zweiten Lichtregime unterteilt, wobei die Leuchte während der Tagphase zumindest zeitweise zur Beleuchtung eingesetzt wird und/oder während der Nachtphase eine Lichtquelle eingesetzt wird, die Licht im Wellenlängenbereich von 500 nm oder mehr und im wesentlichen kein Licht im Wellenlängenbereich unter 500 nm emittiert.

In der Tagphase werden die laktierenden Tiere im überdachten Aufenthaltsraum durch Beleuchtung mit sichtbarem Licht mit ausreichender Helligkeit versorgt. Die Nachtphase kann als Ruhephase ein dunkleres Lichtregime aufweisen, es ist aber in der Regel zweckmäßig, auch in der Nachtphase den überdachten Aufenthaltsraum mit sichtbarem Licht zu beleuchten, um den Tieren eine einfache Orientierung zu ermöglichen.

Das verwendete Lichtregime ist hinsichtlich Intensität und zeitlichen Einsatz prinzipiell beliebig steuerbar. Die jeweiligen Phasen können beliebig verkürzt, verlängert, nach vorne oder hinten verschoben werden. Allerdings ist zu berücksichtigen, dass es sich bei Tieren um biologische Systeme handelt, die träge reagieren und sich nur langsam umstellen. Nach Beginn des erfindungsgemäßen Verfahrens kann daher eine Gewöhnungsphase, z.B. von einigen Tagen, z.B. 30 Tage oder mehr, vorhanden sein, bis der Vitamin-D3-Gehalt in der Milch sich auf ein neues erhöhtes Gleichgewicht einpegelt. Andererseits führt ein Aussetzen der erfindungsgemäßen Bestrahlung für einige Tage nur allmählich zur Rückführung in den ursprünglichen Zustand.

Ungeachtet zeitlich beliebig steuerbarer Lichteinsätze ist es vorteilhaft, dass die Tagphase z.B. etwa 6 bis 22 Stunden, bevorzugt 8 bis 20 Stunden, zweckmäßigerweise etwa 12 bis 21 Stunden und bevorzugt etwa 14 bis 20 Stunden dauert. Eine günstige Dauer beträgt z.B. etwa 17 Stunden plus/minus 1 Stunde oder mehr. Die Nachtphase kann z.B. etwa 2 bis 16 Stunden, zweckmäßigerweise etwa 3 bis 12 Stunden und bevorzugt etwa 4 bis 10 Stunden dauern. Eine besonders günstige Dauer beträgt z.B. etwa 7 Stunden plus/minus 1 Stunde oder weniger.

Die erfindungsgemäße Leuchte wird bevorzugt zur Beleuchtung des überdachten Aufenthaltsraums mit sichtbarem Licht in der Tagphase verwendet. Auf diese Weise liefert die Leuchte nicht nur Strahlung im UV-A- und UV-B-Bereich, sondern auch die Beleuchtung mit sichtbarem Licht. Bevorzugt handelt es sich bei der erfindungsgemäß eingesetzten Lampe um eine Vollspektrumlampe. Das Emissionsspektrum von Vollspektrumlampen ist dem Sonnenlicht am ähnlichsten. Zusätzlich zur erfindungsgemäßen Leuchte können gegebenenfalls übliche Leuchten zur Beleuchtung in der Tagphase eingesetzt werden.

In der Nachtphase werden die Tiere vorzugsweise unter ein Lichtregime gestellt, in der eine Lichtquelle eingesetzt wird, die Licht im Wellenlängenbereich von 500 nm oder mehr und im wesentlichen kein Licht im Wellenlängenbereich unter 500 nm emittiert. Die für die Nachtphase verwendete Lichtquelle emittiert insbesondere Licht der Farbe gelb, orange, amber oder rot oder einer Mischfarbe davon. Die Lichtquelle weist daher im Wellenlängenbereich des sichtbaren Lichts ein Emissionsspektrum auf, das den höchsten Wert mit einer relativen Intensität von 100% bei einer Wellenlänge von 500 nm oder mehr zeigt.

Dass die Lichtquelle im wesentlichen kein Licht mit einer Wellenlänge unter 500 nm emittiert bedeutet dabei insbesondere, dass im Emissionsspektrum des sichtbaren Lichts unter 500 nm jeder messbare Wert, falls überhaupt vorhanden, eine relative Intensität von weniger als 15%, bevorzugt weniger als 10% und besonders bevorzugt unter 5 bzw. unter 3% aufweist. Bevorzugt emittiert die eingesetzte Lichtquelle im wesentlichen kein Licht im Wellenlängenbereich unter 520 nm und bevorzugter unter 540 nm. Besonders bevorzugt emittiert die eingesetzte Lichtquelle kein Licht im Wellenlängenbereich unter 500 nm.

Als Lichtquelle für die Nachtphase können z.B. übliche Lampen, wie z.B. Temperaturstrahler, Kontiniumstrahler, Linienstrahler, Gasentladungslampen, verwendet werden, die einen Monochromator enthalten, so dass im wesentlichen kein Licht mit einer Wellenlänge unter 500 nm emittiert wird. Beispiele für Monochromatoren sind Prismas, Beugungsgitter und optische Filter. Auf diese Weise können z.B. Rotlichtlampen hergestellt werden.

Es werden aber für die Nachtphase bevorzugt Lichtquellen eingesetzt, die keinen Monochromator erfordern. Vorzugsweise wird ein Lumineszenzstrahler als Lichtquelle verwendet. Lumineszenzstrahler können sogenannte Linienstrahler oder monochromatische Strahler sein. Beispiele für Lumineszenzstrahler sind Gasentladungslampen und Leuchtdioden (LED).

Das Emissionsspektrum der Lichtquelle im Wellenlängenbereich des sichtbaren Lichts hat vorzugsweise mindestens ein Maximum über 550 nm, bevorzugter mindestens ein Maximum über 570 nm und noch bevorzugter über 600 nm.

Eine geeignete Lichtquelle ist z.B. eine Natriumdampflampe (NDL). NDL sind Gasentladungslampen, welche monochromatisches gelbes Licht mit einer Wellenlänge von etwa 589 bis 590 nm emittieren. Eine besonders geeignete Lichtquelle für die Nachtphase sind LEDs. Mit LED-Lampen kann der gewünschte Wellenlängenbereich gezielt eingestellt werden und sie besitzen gleichzeitig eine genügend große photopische Wirkung, so dass die Tiere sich bei Beleuchtung mit diesen Lichtquellen gut orientieren können.

Geeignete LEDs sind LED-Lampen mit der Lichtfarbe rot, amber (auch bernsteinfarben oder "superorange") (z.B. Maximum ca. 612 nm), orange (z.B. Maximum ca. 605 nm) oder gelb (z.B. Maximum ca. 585 nm) sowie Mischfarben dieser Spektren. Bevorzugt sind rote Leuchtdioden (z.B. Maximum ca. 630 nm; einschließlich "Ultrarot" bei einem Maximum von ca. 660 nm). Solche LEDs sind handelsüblich und überall erhältlich. Beispiele für im Handel erhältliche LEDs sind z.B. Lumileds® Luxeon red 1 Watt, Lumileds® Luxeon Star/O red 1 Watt oder SOUL R32 red 1 Watt.

Die Lichtquelle für die Nachtphase, insbesondere die LED-Lampe, ist während der Nachtphase in der Regel mindestens 1 Stunde, bevorzugt mindestens 2 Stunden, bevorzugter mindestens 5 Stunden und noch bevorzugter mindestens 6 Stunden im Einsatz.

Überraschenderweise kann die Umgebung der Tiere mit den genannten Lichtquellen in der Nachtphase relativ hell beleuchtet werden. Die Beleuchtungsstärke, die durch die in der Nachtphase verwendeten Lichtquellen erhalten wird, kann bevorzugt mehr als 50 lux betragen, es sind aber auch Beleuchtungstärken unter 50 lux möglich. Prinzipiell können hohe Beleuchtungsstärken in der Nachtphase verwendet werden, in der Regel ist sie aber z.B. nicht mehr als 150 lux, bevorzugter nicht mehr als 100 lux. Die Beleuchtungsstärke in der Nachtphase ist z.B. um mindestens 50 lux, bevorzugt um mindestens 100 lux, kleiner als die Beleuchtungsstärke in der Tagphase. Für die Messung der Beleuchtungsstärke wird auf die vorstehenden Angaben verwiesen.

Das Verfahren umfasst ferner das Melken der Tiere. Das Melken kann wie üblich durchgeführt werden. Es sind hier keine Änderungen erforderlich. Die Tiere können z.B. einmal, zweimal, dreimal oder häufiger pro Tag gemolken werden. Wie üblich ist ein Melken in der Tagphase, z.B. morgens und abends oder einmal in der Tagphase, gewöhnlich zweckmäßig. Es kann aber auch alternativ oder zusätzlich in der Nachtphase gemolken werden.

Es versteht sich, dass die Bestrahlung der Tiere mit der mindestens einer Leuchte umfassend eine Lampe, die UV-A-Strahlung und UV-B-Strahlung emittiert, gemäß der Erfindung unabhängig ist vom Zeitpunkt des Melkvorgangs. Insbesondere kann das Melken zu Zeitpunkten stattfinden, bei denen diese Bestrahlung durchgeführt wird oder diese Bestrahlung nicht durchgeführt wird.

Natives Vitamin D3 bindet sich vornehmlich an Fettmoleküle und Proteine der Milch. Durch Fettreduktion kann sich daher auch der Vitamin D3-Gehalt vermindern. Natives Vitamin D3 ist temperaturunempfindlich.

Normale, im Handel erhältliche Milch, die nach dem üblichen Verfahren hergestellt wird, enthält Vitamin D-Gehalte von ca. 0,1 µg/100 ml Milch (It. Bundeslebensmittelschlüssel, Stand 2014). Zur Herstellung von handelsüblicher Milch werden im Gegensatz zum erfinderischen Verfahren in den Ställen für die Beleuchtung tagsüber herkömmliche Lampen, z.B. Glühbirnen, Neonröhren oder Natriumdampflampen eingesetzt. Eine Bestrahlung mit UV-B-Anteil erfolgt nicht.

Der Vitamin D3-Gehalt in der Milch wird standardmäßig durch eine HPLC/UV-Methode bestimmt. Alternativ kann der Vitamin D3-Gehalt auch mittels ELISA (Enzyme Linked Immunosorbent Assay) bestimmt werden. Mit Hilfe des ELISA kann der Gehalt an Vitamin D3 in einer Probe wie Milch auch zuverlässig bestimmt werden und wird in einigen Ländern als Standard verwendet. Dies wurde durch Testmessungen in unabhängigen und zertifizierten Analyselaboratorien bestätigt.

Die nach dem erfindungsgemäßen Verfahren hergestellte Milch enthält einen deutlich gesteigerten Vitamin D3-Gehalt. Sie kann z.B. einen Vitamin-D3-Gehalt von mehr als 0,5 µg/100 ml Milch, bevorzugt mehr als 1 µg/100 ml Milch und besonders bevorzugt mehr als 1,5 µg/100 ml Milch aufweisen, wobei z.B. Gehalte von 1,5 bis 4 µg/100 ml und mehr erhalten werden können. Bei dem Vitamin D3 der Milch handelt es sich um natives Vitamin D3. Die angegebenen Werte beziehen sich auf die Bestimmung mittels ELISA oder HPLC/UV, wobei der Vitamin D3-Gehalt bestimmt wird.

Durch das erfindungsgemäße Verfahren wird der native Vitamin D3-Gehalt der Milch gesteigert. Unter einen gesteigerten nativen Vitamin D3-Gehalt wir hierbei insbesondere ein Vitamin D3-Gehalt von mindestens 0,5 µg/100 ml Milch, bevorzugt mehr als 1 µg/100 ml Milch und besonders bevorzugt mehr als 1,5 µg/100 ml Milch verstanden.

Durch das erfindungsgemäße Verfahren kann auch die Milchleistung pro Tier gesteigert werden im Vergleich zu einem gleichen Verfahren, bei dem aber statt der Lampe, die UV-B-Strahlung emittiert, eine normale Lampe ohne Emission im UV-B-Bereich verwendet wird. Hierfür ist eine durch das Licht erzeugte starke Helligkeit durch die Lampe vorteilhaft, welche z. B. mit einer Vollspektrumlampe oder einer weißen LED erzeugt werden kann. Geeignete Bestrahlungsstärke sind z.B. mindestens 50 Lux, bevorzugter mindestens 80 Lux, besonders bevorzugt mindestens 100 Lux. Die tägliche Bestrahlung in der Tagphase kann hierfür z.B. mindestens 4 Stunden, bevorzugt mindestens 10 Stunden und besonders bevorzugt mindestens 16 Stunden erfolgen. Die Milchleistung pro Tier kann z.B. um mindestens 2%, bevorzugt mindestens 4% erhöht sein.

Das Hormon Melatonin ist für Organismen wichtig, weswegen eine geeignete Quelle hierfür wünschenswert wäre. Es ist bekannt, dass das Hormon Melantonin in der Milch von laktierenden Tieren vorhanden ist. Die Mengen sind aber normalerweise relativ gering, so dass gewöhnliche Milch nicht als Quelle für Melatonin geeignet ist. Es hat sich gezeigt, dass bei einer bevorzugten Ausführungsform des erfindungsgemäßen Verfahrens auch Milch mit einem deutlich erhöhten Anteil an Melatonin erhalten werden kann.

Bei der vorstehend beschriebenen bevorzugten Ausführungsform des erfindungsgemäßen Verfahrens, bei dem der Tagesrhythmus der Tiere in eine Tagphase unter einem ersten Lichtregime und in eine Nachtphase unter einem zweiten Lichtregime unterteilt wird, wobei die Leuchte während der Tagphase zumindest zeitweise zur Beleuchtung eingesetzt wird und während der Nachtphase eine Lichtquelle eingesetzt wird, die Licht im Wellenlängenbereich von 500 nm oder mehr und im wesentlichen kein Licht im Wellenlängenbereich unter 500 nm emittiert, enthält die Milch in der Nachtphase nämlich nicht nur einen hohen nativen Vitamin D3-Gehalt, sondern auch einen hohen Melatoningehalt. Ein Melken der Tiere in der Nachtphase oder kurz nach der Nachtphase (z.B. bis zu einer Stunde nach der Nachtphase) führt daher zu einer Milch mit einem hohen nativen Vitamin D3-Gehalt und einem erhöhten Melatoningehalt. Das Melatonin in der Milch wird in der Tagphase durch die Tagesbeleuchtung wieder relativ rasch abgebaut, so dass das Melken hierfür bevorzugt kurz nach der Nachtphase, bevorzugter in der Nachtphase erfolgt. Dies schließt naturgemäß ein Melken auch in der Tagphase nicht aus, die daraus erhaltene Milch weist allerdings keine erhöhten Melatoninkonzentrationen auf.

Die Erfindung betrifft auch einen überdachten Aufenthaltsraum, zur Haltung von laktierenden Tieren, wobei in dem überdachten Aufenthaltsraum mindestens eine Leuchte angebracht ist, die eine Lampe, die UV-A-Strahlung und UV-B-Strahlung emittiert, umfasst, wobei die Leuchte vorzugsweise eine Lampenabdeckung umfasst, die für UV-B-Strahlung bei einer Wellenlänge von 300 nm durchlässig ist.

### Beispiele

### Vergleichsbeispiele 1 bis 4

In zwei Pilotbetrieben (Betrieb I mit 1100 Milchkühen, Betrieb II mit 420 Milchkühen) wurde die produzierte Milch im Hinblick auf die Vitamin-D-Konzentration untersucht. Die Tiere wurden in Ställen mit stressvermindernden Freilaufsystemen gehalten, bei denen sie ihre Fress-, Trink- und Liegeplätze frei aufsuchen konnten. Zudem wurden den Tieren gras- und kräuterbasierte Futterrationen zur Verfügung gestellt. Die Tiere wurden tagsüber und teilweise während der Nachtphase gemolken. Die Tiere wurden in folgendem Tag/Nachtrhythmus gehalten:
Die Dauer der Tagphase betrug etwa 16 Stunden. Die Dauer der Nachtphase betrug etwa 8 Stunden. In der Tagphase wurden die Tiere mit handelsüblichen Leuchtstoffröhren, die ca. 3m über dem Stallboden angeordnet waren, mit sichtbarem Licht versorgt. Die Leuchtstoffröhren emittierten keine Strahlung im UV-B-Bereich. In der Nachtphase wurden beide Tiergruppen unter einem Lichtregime mit Licht mit einer Wellenlänge nicht unter 500 nm gehalten. Hierfür wurden rote LED-Lampen eingesetzt.

Nach einer Gewöhnungsphase wurde im Betrieb I an unterschiedlichen Tagen gesammelte Milch auf den Vitamin-D-Gehalt (Vitamin D3) mittels HPLC/UV untersucht (Vergleichsbeispiele 1 bis 4). Die Ergebnisse sind in Tabelle 1 wiedergegeben. Die Werte stimmen mit den für handelsübliche Milch angegebenen Werten für den Vitamin-D-Gehalt überein (ca. 0,1 µg/100 ml Milch It. Bundeslebensmittelschlüssel).

### Beispiele 1 bis 7

Der Betrieb in den beiden Pilotbetrieben wurde in gleicher Weise fortgesetzt, außer dass die handelsüblichen Leuchtstoffröhren für die Beleuchtung in der Tagphase durch Leuchten mit speziellen Leuchtstofflampen mit einer ähnlichen Belichtungsstärke im sichtbaren Bereich ersetzt wurden. Tagsüber wurde die Tiere so künstlichem Licht mit sonnenlichtähnlichem Lichtspektrum ausgesetzt. Für das Licht wurden als Vollspektrumlampen spezielle Leuchtstofflampen eingesetzt, die folgendes Emissionsspektrum aufwiesen:

| | |
|---|---|
| VIS-Anteil (380-780 nm) | ca. 90,85% |
| UV-A-Anteil (315-380 nm) | ca. 9 % |
| UV-B-Anteil (280-315nm): | ca. 0,15% |
| UV-C-Anteil (200-280 nm): | 0 %. |

Es wurde täglich mit 2x58 Watt-Vollspektrum-Leuchtstofflampen ca. 3 m über dem Stallboden beleuchtet. Die Leuchtstofflampen waren von der Schutzart IP 65. Die Leuchtstofflampen umfassten eine Lampenabdeckung. Die Lampenabdeckung war aus PMMA (Plexiglas 6N von Degussa) mit einer Wanddicke von 3 mm gebildet. Die Figur zeigt eine Transmissionskurve des Plexiglases von dieser Wanddicke. Bei einer Wellenlänge von 300 nm beträgt der Transmissionsgrad ca. 70%.
Nach einer Gewöhnungsphase unter diesem Tag/Nachtrhythmus wurde an verschiedenen Tagen die Milch auf den Vitamin-D-Gehalt (Vitamin D3) mittels ELISA untersucht (Beispiele 1 bis 7). Die auf diese Art und Weise erzeugte Milch enthielt ein Vielfaches an Vitamin-D im Vergleich zu Milch aus der herkömmlichen Milchproduktion. Die Vitamin-D-Gehalte lagen bei 1,63 bis 1,92 µg/100 ml Milch. In Tabelle 1 sind die Ergebnisse der betreffenden Proben, welche aus den jeweiligen Milchtanks bzw. Mischproben gezogen wurden, wiedergegeben. Die Tanks wie auch die Mischproben umfassen die gesammelte Milch mehrerer Kühe. Der durchschnittliche Fettgehalt der Milchproben betrug ca. 4%.

**Tabelle 1: Vitamin D-Gehalte in der Milch**

| Probe - Nr. | | Vitamin-D3-Gehalt µg/100 ml Milch |
|---|---|---|
| Vgl.-bsp. 1 | Betrieb I Mischprobe | < 0,2* |
| Vgl.-bsp. 2 | Betrieb I Mischprobe | < 0,2* |
| Vgl.-bsp. 3 | Betrieb I Mischprobe | < 0,2* |
| Vgl.-bsp. 4 | Betrieb I Mischprobe | < 0,2* |
| Bsp. 1 | Betrieb II Mischprobe | 1,63 |
| Bsp. 2 | Betrieb II Mischprobe | 1,87 |
| Bsp. 3 | Betrieb I Tank 1 | 1,91 |
| Bsp. 4 | Betrieb I Tank 2 | 1,92 |
| Bsp. 5 | Betrieb I Tank 3 | 1,90 |
| Bsp. 6 | Betrieb I Mischprobe | 1,75 |
| Bsp. 7 | Betrieb I Mischprobe | 1,83 |

| | | |
|---|---|---|
| *unterhalb der Nachweisgrenze | | |

Nach einem einjährigen Betrieb gemäß dem erfindungsgemäßen Verfahren konnten auch folgende Phänomene im Vergleich zum Zeitraum vor der Einführung des Verfahrens beobachtet werden:
- die Krankheitsfälle und Reproduktionsraten der Tiere sanken,
- die Tierarztkosten des Betriebs sanken,
- die Milchleistung pro Tier stieg um über 5%.

### Beispiele 8 bis 12

Der Betrieb in den beiden Pilotbetrieben wurde in gleicher Weise wie in den Beispielen 1-7 fortgesetzt, außer dass die in den Beispielen 1-7 eingesetzten Leuchtstofflampen für die Beleuchtung in der Tagphase durch Leuchten mit speziellen Leuchtstofflampen mit einer ähnlichen Belichtungsstärke im sichtbaren Bereich ersetzt wurden. Tagsüber wurde die Tiere so künstlichem Licht mit sonnenlichtähnlichem Lichtspektrum ausgesetzt. Für das Licht wurden als Vollspektrumlampen spezielle Leuchtstofflampen mit einem noch höheren UV-B-Anteil eingesetzt, die als Leuchtstoffe eine Mischung von ca. 36 % SBPE, ca. 50 % SMS, ca. 13 % BAC und ca. 1 % LAP enthielten. Die Leuchtstofflampen wiesen folgendes Emissionsspektrum auf:

| | |
|---|---|
| VIS-Anteil (380-780 nm) | ca. 90% |
| UV-A-Anteil (315-380 nm) | ca. 9 % |
| UV-B-Anteil (280-315nm): | ca. 1% |
| UV-C-Anteil (200-280 nm): | 0 %. |

Es wurde täglich mit 2x58 Watt-Vollspektrum-Leuchtstofflampen ca. 3 m über dem Stallboden beleuchtet. Die Leuchtstofflampen waren von der Schutzart IP 65. Die Leuchtstofflampen umfassten eine Lampenabdeckung. Die Lampenabdeckung war aus PMMA (Plexiglas 6N von Degussa) mit einer Wanddicke von 3 mm gebildet. Die Figur zeigt eine Transmissionskurve des Plexiglases von dieser Wanddicke. Bei einer Wellenlänge von 300 nm beträgt der Transmissionsgrad ca. 70%.
Nach einer Gewöhnungsphase unter diesem Tag/Nachtrhythmus wurde an verschiedenen Tagen die Milch auf den Vitamin-D-Gehalt (Vitamin D3) mittels ELISA untersucht (Beispiele 8 bis 12). Die auf diese Art und Weise erzeugte Milch enthielt ein Vielfaches an Vitamin-D im Vergleich zu Milch aus der herkömmlichen Milchproduktion. Die Vitamin-D-Gehalte lagen bei 2,34 bis 2,67 µg/100 ml Milch. In Tabelle 2 sind die Ergebnisse der betreffenden Proben, welche aus den jeweiligen Milchtanks gezogen wurden, wiedergegeben. Die Tanks umfassen die gesammelte Milch mehrerer Kühe. Der durchschnittliche Fettgehalt der Milchproben betrug ca. 4%.

**Tabelle 2: Vitamin D-Gehalte in der Milch**

| Probe - Nr. | | Vitamin-D3-Gehalt µg/100 ml Milch |
|---|---|---|
| Bsp. 8 | Betrieb I Tank 1 | 2,67 |
| Bsp. 9 | Betrieb I Tank 2 | 2,67 |
| Bsp. 10 | Betrieb I Tank 3 | 2,65 |
| Bsp. 11 | Betrieb I Tank 4 | 2,55 |
| Bsp. 12 | Betrieb I Tank 5 | 2,34 |

Nach einem einjährigen Betrieb gemäß dem erfindungsgemäßen Verfahren konnten auch folgende Phänomene im Vergleich zum für die Vergleichsbeispiele 1 bis 4 verwendetenVerfahren beobachtet werden:
- die Krankheitsfälle und Reproduktionsraten der Tiere sanken,
- die Tierarztkosten des Betriebs sanken,
- die Milchleistung pro Tier stieg um über 5%.

## Patentansprüche

1. Verfahren zur Herstellung von Milch mit hohem nativem Vitamin D3-Gehalt umfassend die Bestrahlung eines oder mehrerer laktierende Tiere in einem überdachten Aufenthaltsraum mit mindestens einer Leuchte umfassend eine Lampe, die UV-A-Strahlung und UV-B-Strahlung emittiert, und das Melken der Tiere, wobei die Lampe eine Vollspektrumlampe ist und der Anteil an UV-B-Strahlung im Emissionsspektrum der Lampe mindestens 0,2% und weniger als 5% der Bestrahlungsstärke der Lampe beträgt.

2. Verfahren nach Anspruch 1, wobei die Leuchte eine Lampenabdeckung umfasst, die für UV-B-Strahlung bei einer Wellenlänge von 300 nm durchlässig ist, wobei die Lampenabdeckung bevorzugt aus Quarzglas, Borosilikatglas, Polymethylmethacrylat, Polymethylpenten, Fluoriertes-Ethylen-Propylen oder Polytetrafluorethylen gebildet ist.

3. Verfahren nach Anspruch 1 oder 2 zur Steigerung des nativen Vitamin D3-Gehalts der Milch und/oder zur Steigerung der Milchleistung pro Tier.

4. Verfahren nach irgendeinem der Ansprüche 1 bis 3, wobei das Emissionsspektrum der Lampe sichtbares Licht umfasst und/oder die Lampe einen Farbwiedergabeindex von größer 40, bevorzugt größer 70, aufweist.

5. Verfahren nach irgendeinem der Ansprüche 1 bis 4, wobei der Anteil an Strahlung im VIS-Bereich im Emissionsspektrum der Lampe mindestens 70% beträgt.

6. Verfahren nach irgendeinem der Ansprüche 1 bis 5, wobei der Anteil an UV-B-Strahlung im Emissionsspektrum der Lampe mindestens 0,5% der Bestrahlungsstärke der Lampe beträgt und wobei der Anteil an UV-B-Strahlung im Emissionsspektrum der Lampe weniger als 5% der Bestrahlungsstärke der Lampe beträgt.

7. Verfahren nach irgendeinem der Ansprüche 1 bis 6, wobei die Lampe ausgewählt ist aus einer Gasentladungslampe, einer Leuchtstofflampe oder einer Halogen-Metalldampflampe.

8. Verfahren nach Anspruch 7, wobei die Leuchtstofflampe eine Niederdruckentladungslampe ist.

9. Verfahren nach irgendeinem der Ansprüche 1 bis 8, wobei die Lampe eine Leuchtstofflampe ist, die Strontium-Borophosphat:Eu, und/ oder Strontium-Magnesium-Phosphat:Sn, und/oder Barium-Magnesium-Aluminat:Ce und/oder Lanthan-Phosphat: Ce als Leuchtstoffe umfasst.

10. Verfahren nach irgendeinem der Ansprüche 1 bis 9, wobei der Tagesrhythmus der Tiere in eine Tagphase unter einem ersten Lichtregime und in eine Nachtphase unter einem zweiten Lichtregime unterteilt wird, wobei die Leuchte während der Tagphase zumindest zeitweise zur Beleuchtung eingesetzt wird und/oder während der Nachtphase eine Lichtquelle eingesetzt wird, die Licht im Wellenlängenbereich von 500 nm oder mehr und im wesentlichen kein Licht im Wellenlängenbereich unter 500 nm emittiert.

11. Verfahren nach Anspruch 10, wobei die für die Nachtphase verwendet Lichtquelle eine LED-Lampe oder eine Natriumdampflampe ist.

12. Verfahren nach einem der Ansprüche 1 bis 11, wobei das Tier ein Schaf, eine Ziege oder eine Kuh ist, wobei das Tier insbesondere eine Kuh ist.

13. Verfahren nach einem der Ansprüche 1 bis 12, wobei die Leuchte staubdicht und gegen Strahlwasser geschützt ist, wobei die Leuchte bevorzugt von der Schutzart IP 65 ist.

14. Verfahren nach einem der Ansprüche 2 bis 13, wobei die Lampenabdeckung an einem Gehäuse der Leuchte angebracht ist und zwischen der Lampenabdeckung und dem Gehäuse eine Dichtung mit darauf aufgebrachtem Talkum angeordnet ist.

15. Überdachter Aufenthaltsraum, insbesondere Stall, zur Haltung von laktierenden Tieren, wobei in dem Aufenthaltsraum mindestens eine Leuchte angebracht ist, die eine Lampe, die UV-A-Strahlung und UV-B-Strahlung emittiert, umfasst, wobei die Lampe eine Vollspektrumlampe ist, **dadurch gekennzeichnet, dass** der Anteil an UV-B-Strahlung im Emissionsspektrum der Lampe mindestens 0,2% und weniger als 5% der Bestrahlungsstärke der Lampe beträgt, wobei die Leuchte vorzugsweise eine Lampenabdeckung umfasst, die für UV-B-Strahlung bei einer Wellenlänge von 300 nm durchlässig ist.

## Claims

1. A method for the production of milk with a high native vitamin D3 content, comprising the irradiation of one or more lactating animals in a roofed enclosure having at least one luminaire comprising a lamp which emits UV-A radiation and UV-B radiation, and the milking of the animals, wherein the lamp is a full-spectrum lamp and the proportion of UV-B radiation in the emission spectrum of the lamp is at least 0.2% and less than 5% of the irradiation intensity of the lamp.

2. The method according to claim 1, the luminaire comprising a lamp cover which is permeable to UV-B radiation of a wavelength of 300 nm, the lamp cover preferably being formed of quartz glass, borosilicate glass, polymethyl methacrylate, polymethylpentene, fluorinated ethylene propylene or polytetrafluoroethylene.

3. The method according to claim 1 or claim 2 for increasing the native vitamin D3 content of the milk and/or for increasing the milk yield per animal.

4. The method according to any one of claims 1 to 3, the emission spectrum of the lamp comprising visible light and/or the lamp having a color rendering index of greater than 40, preferably greater than 70.

5. The method according to any one of claims 1 to 4, the proportion of radiation in the VIS range in the emission spectrum of the lamp being at least 70%.

6. The method according to any one of claims 1 to 5, the proportion of UV-B radiation in the emission spectrum of the lamp being at least 0.5% of the irradiation intensity of the lamp and the proportion of UV-B radiation in the emission spectrum of the lamp being less than 5% of the irradiation intensity of the lamp.

7. The method according to any one of claims 1 to 6, the lamp being selected from among a gas discharge lamp, a fluorescent lamp or a halogen metal vapor lamp.

8. The method according to claim 7, the fluorescent lamp being a low-pressure discharge lamp.

9. The method according to any one of claims 1 to 8, the lamp being a fluorescent lamp which comprises strontium borophosphate:Eu and/or strontium magnesium phosphate:Sn and/or barium magnesium aluminate:Ce and/or lanthanum phosphate:Ce as luminescent substances.

10. The method according to any one of claims 1 to 9, the daily rhythm of the animals being divided into a day phase with a first light regimen and a night phase with a second light regimen, the luminaire being employed for illumination purposes at least for some time during the day phase and/or a light source being employed during the night phase which emits light in the wavelength range of 500 nm or more and essentially no light in the wavelength range of below 500 nm.

11. The method according to claim 10, wherein the light source used for the night phase is an LED lamp or a sodium vapor lamp.

12. The method according to any one of claims 1 to 11, the animal being a sheep, a goat or a cow, the animal being especially a cow.

13. The method according to any one of claims 1 to 12, the luminaire being dustproof and protected against water jets, the luminaire preferably having a type of protection of IP 65.

14. The method according to any one of claims 2 to 13, the lamp cover being fixed to a housing of the luminaire, and a seal with talcum applied thereto being arranged between the lamp cover and the housing.

15. A roofed enclosure, especially a building for livestock, for keeping lactating animals, at least one luminaire being fixed in the enclosure which comprises a lamp which emits UV-A radiation and UV-B radiation, wherein the lamp is a full-spectrum lamp, **characterized in that** the proportion of UV-B radiation in the emission spectrum of the lamp is at least 0.2% and less than 5% of the irradiation intensity of the lamp, wherein the luminaire preferably comprising a lamp cover which is permeable to UV-B radiation at a wavelength of 300 nm.

## Revendications

1. Procédé de production de lait à haute teneur en vitamine D3 native, comprenant les étapes consistant à irradier une ou plusieurs bêtes allaitantes dans un enclos couvert avec au moins un luminaire comprenant une lampe qui émet des rayons UV-A et des rayons UV-B, et traire les bêtes, la lampe étant une lampe à spectre complet et la proportion de rayons UV-B dans le spectre d'émission de la lampe étant d'au moins 0,2 % et étant inférieure à 5 % de l'intensité d'irradiation de la lampe.

2. Procédé selon la revendication 1, dans lequel le luminaire comprend un hublot de lampe, qui laisse passer les rayons UV-B d'une longueur d'ondes de 300 nm, le hublot de lampe étant constitué de préférence de verre de quartz, de verre de borosilicate, de polyméthacrylate de méthyle, de polyméthylpentène, d'éthylène-propylène fluoré ou de polytétrafluoroéthylène.

3. Procédé selon la revendication 1 ou 2, pour augmenter la teneur en vitamine D3 native du lait et/ou pour augmenter le rendement laitier par bête.

4. Procédé selon l'une quelconque des revendications 1 à 3, dans lequel le spectre d'émission de la lampe comprend la lumière visible et/ou la lampe présente un indice de rendu des couleurs supérieur à 40, de préférence supérieur à 70.

5. Procédé selon l'une quelconque des revendications 1 à 4, dans lequel la proportion de rayons dans le domaine VIS dans le spectre d'émission de la lampe est d'au moins 70 %.

6. Procédé selon l'une quelconque des revendications 1 à 5, dans lequel la proportion de rayons UV-B dans le spectre d'émission de la lampe est d'au moins 0,5 % de l'intensité d'irradiation de la lampe et dans lequel la proportion de rayons UV-B dans le spectre d'émission de la lampe est inférieure à 5 % de l'intensité d'irradiation de la lampe.

7. Procédé selon l'une quelconque des revendications 1 à 6, dans lequel la lampe est choisie parmi une lampe à décharge de gaz, une lampe fluorescente ou une lampe aux halogénures métalliques.

8. Procédé selon la revendication 7, dans lequel la lampe fluorescente est une lampe à décharge basse pression.

9. Procédé selon l'une quelconque des revendications 1 à 8, dans lequel la lampe est une lampe fluorescente qui comprend du borophosphate de strontium:Eu, et/ou du phosphate de strontium-magnésium:Sn, et/ou de l'aluminate de baryum-magnésium:Ce et/ou du phosphate de lanthane:Ce comme substances fluorescentes.

10. Procédé selon l'une quelconque des revendications 1 à 9, dans lequel le rythme quotidien des bêtes est subdivisé en une phase diurne sous un premier régime lumineux et en une phase nocturne sous un deuxième régime lumineux, le luminaire étant mis en oeuvre pendant la phase diurne au moins par moments pour l'éclairage et/ou une source de lumière émettant de la lumière dans la plage de longueurs d'ondes de 500 nm ou plus et sensiblement pas de lumière dans la plage de longueurs d'ondes inférieure à 500 nm étant mise en oeuvre pendant la phase nocturne.

11. Procédé selon la revendication 10, dans lequel la source de lumière utilisée pour la phase nocturne est une lampe DEL ou une lampe à vapeur de sodium.

12. Procédé selon l'une des revendications 1 à 11, dans lequel la bête est une brebis, une chèvre ou une vache, la bête étant en particulier une vache.

13. Procédé selon l'une des revendications 1 à 12, dans lequel le luminaire est conçu pour être étanche à la poussière et aux projections d'eau, le luminaire étant de préférence du type de protection IP 65.

14. Procédé selon l'une des revendications 2 à 13, dans lequel le hublot de lampe est placé sur un boîtier du luminaire et un joint appliqué de talc est disposé entre le hublot de lampe et le boîtier.

15. Enclos couvert, en particulier étable, pour abriter des bêtes allaitantes, dans lequel est disposé au moins un luminaire qui comprend une lampe émettant des rayons UV-A et des rayons UV-B, la lampe étant une lampe à spectre complet, **caractérisé en ce que** la proportion de rayons UV-B dans le spectre d'émission de la lampe est d'au moins 0,2 % et est inférieure à 5 % de l'intensité d'irradiation de la lampe, le luminaire comprenant de préférence un hublot de lampe qui laisse passer les rayons UV-B d'une longueur d'ondes de 300 nm.
